# EUROPEAN PATENT SPECIFICATION

(11) **EP 0 756 617 B1**
(45) Date of publication and mention of the grant of the patent: **03.03.1999**
(21) Application number: 94903787.3
(22) Date of filing: 13.12.1993
(51) Int. Cl.: C10L 1/22, C07C 255/15, C07C 255/34, C07C 217/42, C07C 217/48

(54) **GASOLINE COMPOSITION**
BENZINZUSAMMENSETZUNG
COMPOSITION D'ESSENCE

(43) Date of publication of application: 05.02.1997
(73) Proprietor: SHELL INTERNATIONALE RESEARCH MAATSCHAPPIJ B.V., 2596 HR Den Haag (NL)
(72) Inventor: JOHNSON, Thomas, Howard, Houston, TX 77083 (US)
(86) International application number: EP9303556
(87) International publication number: WO9516764

(56) References cited:
- EP-A- 0 018 473
- EP-A- 0 289 785
- EP-A- 0 422 859
- EP-A- 0 440 508
- EP-A- 0 568 873
- FR-A- 2 333 853
- GB-A- 2 175 900
- US-A- 2 824 121
- US-A- 4 247 301
- US-A- 4 670 021

## Description

The present invention relates to gasoline compositions and gasoline additive concentrates containing certain dinitrile compounds and their derivatives useful as additives for reducing intake valve deposit formation.

Gasoline compositions have traditionally been formulated to improve the performance of carburetor and throttle body injected engines. Beginning in about 1984, electronic port fuel injected engines were commonly introduced by automobile manufacturers. Shortly thereafter, in about 1985, problems began to be reported with intake valve deposits in electronic port fuel injected engines, which problems are characterized by hard starting, stalls, and stumbles during acceleration and rough engine idle.

Conventional commercial gasoline additives contain nitrogen. The nitrogens are attached to a polymer, and the nitrogens are separated by two or three carbons. This structure provides a potential for chelation. However, steric hinderance reduces chelatability in conventional additives since the polymer is attached through one of the nitrogen atoms.

Without limiting the invention by theories of operation, it has now been discovered that chelatability is advantageous for its deposit reducing tendencies. Accordingly, it would be desirable to have a new gasoline additive which has increased chelatability and thus reduces or eliminates undesirable intake valve deposits in electronic port fuel injected engines. Also, since some carburetor and throttle body injector engines will still be in use for the foreseeable future, it would be desirable if gasoline containing such an additive could also be compatible with these engines.

Therefore, in accordance with the present invention, there is provided a gasoline composition comprising a major amount (more than 50%w based on total composition of a gasoline and a minor amount, e.g. from 5 to 1000, preferably from 50 to 500, more preferably from 75 to 350 and most preferably from 75 to 250 parts per million by weight (ppmw) based on the total composition, of a compound of the general formula: 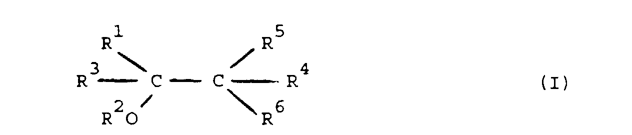 wherein R¹ represents a hydrogen atom or an alkyl, aryl or alkaryl group; R²O is a residue of a polyether mono-ol or a hydrocarbyl mono-ol of formula R²OH; and R³ and R⁴ each represent a hydrogen atom or together represent a carbon-carbon single bond, wherein when R³ and R⁴ each represent a hydrogen atom, then each of R⁵ and R⁶ independently represents a group -CH₂N(R⁷)₂ in which each R⁷ independently represents a hydrogen atom or an optionally substituted alkyl group, and when R³ and R⁴ together represent a carbon-carbon single bond, then R⁵ and R⁶ each represent a cyano group.

In this specification, unless otherwise stated, an alkyl group or an alkyl moiety in an alkaryl group may be linear or branched and preferably contains up to 20, more preferably up to 12, still more preferably up to 10, and most preferably up to 6, carbon atoms. A cycloalkyl group may contain from 3 to 8, preferably 3 to 6, carbon atoms. An aryl group may be any aromatic group, especially a phenyl or naphthyl group. An alkaryl group is an aryl group substituted by one or more, preferably one to two, alkyl groups. A heterocyclyl group may be any saturated or unsaturated ring system, e.g. a C₅-C₇ ring system, containing at least one heteroatom selected from oxygen, nitrogen and sulphur, 5- and 6-membered rings being especially preferred, e.g. a tetrahydrofuranyl, furanyl, piperidinyl, pyridinyl, tetrahydrothiophenyl or thiophenyl (thienyl) group.

It is preferred in formula I that R¹ represents a hydrogen atom, a C₁-C₂₀ alkyl group, or a phenyl or naphthyl group optionally substituted by one or more C₁-C₆ alkyl groups.

More preferably, R¹ represents a hydrogen atom, a C₁-C₁₀ alkyl group, or a phenyl or naphthyl group optionally substituted by one or more, e.g. up to 3, C₁-C₄ alkyl groups.

Still more preferably, R¹ represents a hydrogen atom, a C₁-C₆ alkyl group or a phenyl group. R¹ is especially a hydrogen atom.

R²O is a residue of a polyether mono-ol or a hydrocarbyl mono-ol of formula R²OH.

When R²OH is a hydrocarbyl mono-ol, the hydrocarbyl moiety may be aliphatic or aromatic, linear or branched and preferably contains up to 400 carbon atoms, more preferably up to 200 carbon atoms, e.g. from 50 to 150 carbon atoms.

R²OH is preferably an oil soluble polyether mono-ol of the formula R₁-O-(R₂O)ₙ-H wherein R₁ represents an aliphatic, cycloaliphatic or aromatic hydrocarbon radical containing up to 40 carbon atoms, each moiety R₂, which may be the same or different, represents an alkylene radical containing up to 16, preferably up to 12, carbon atoms, and n is an integer of at least 7.

R²OH is more preferably of the formula R₁O-(EO)ₐ-(PO)_{b}-(BO)_{c}-(XO)_{d}-H wherein R₁ represents an aliphatic, cycloaliphatic or aromatic hydrocarbon radical containing up to 40 carbon atoms, and wherein E is an ethylene moiety, P is a 1,2-propylene moiety, B is a butylene moiety, preferably a 1,2-butylene moiety, X is an alkylene moiety, preferably a 1,2-alkylene moiety, having from 5 to 16 carbon atoms; a, b, c and d are each from 0 to 100, and the sum of a, b, c and d is at least 7. In this form of R²OH, a, b and d each are preferably 0 and c is from 10 to 40. More preferably, R₁O is a moiety resulting from a ring opening of alpha-butylene oxide (1,2-epoxybutane).

In another preferred form R²O is a residue of a polyether mono-ol where R₁ is preferably a butyl or a C₁₂ to C₁₈ alkyl group and n is from 10 to 40, i.e., C₁₂H₂₃O - C₁₈H₃₇O(-CH₂-CH(C₂H₅)O-)ₙH; or C₄H₉O(-CH₂-CH(C₂H₅)O-)ₙH, where n is from 10 to 40, and mixtures thereof. Of this group R²O more preferably is derived from C₄H₉O(-CH₂-CH(C₂H₅)O-)ₙH, where n is from 10 to 40.

Preferably, the polyether mono-ol has a number average molecular weight (Mₙ) not more than 5000. More preferably, the polyether mono-ol has a number average molecular weight in the range from 500 to 3000, still more preferably from 500 to 2500 and especially from 1000 to 2000. Where R²O is derived from C₄H₉O(-CH₂-CH(C₂H₅)O-)ₙH, R²O preferably has a number average molecular weight in the range from 1900 to 2100.

It is preferred in formula I that R³ and R⁴ each represent a hydrogen atom, and each of R⁵ and R⁶ independently represents a group -CH₂N(R⁷)₂ as defined above.

Preferably each R⁷ independently represents a hydrogen atom or a C₁-C₂₀ alkyl group optionally substituted by one or more, preferably one or two, substituents selected from halogen atoms (e.g. chlorine atoms), nitro, hydroxyl, cycloalkyl, alkoxy, haloalkoxy, amino, dialkylamino, formyl, alkoxycarbonyl, carboxyl, alkanoyl, alkylthio, alkylsulphinyl, alkylsulphonyl, carbamoyl, alkylamido, aryl, alkaryl and heterocyclyl groups. When any of the foregoing substituents contain an alkyl moiety, this may be linear or branched and may contain up to 12, preferably up to 6, and especially up to 4, carbon atoms.

More preferably each R⁷ independently represents a hydrogen atom or a C₁-C₁₀ alkyl group and is especially a hydrogen atom.

A particularly preferred sub-group of compounds of formula I is that in which R¹ represents a hydrogen atom; R²O is a residue of a polyether mono-ol of formula R₁-O-(R₂O)ₙ-H wherein R₁ represents a butyl group, R₂ represents a C₄ alkylene radical, preferably a 1,2-butylene radical, and n is an integer from 10 to 30; and either R³ and R⁴ each represent a hydrogen atom and R⁵ and R⁶ each represent a group -CH₂NH₂, or R³ and R⁴ together represent a carbon-carbon single bond and R⁵ and R⁶ each represent a cyano group.

When used in a gasoline composition the compound of formula I is conveniently present in the gasoline in a quantity of 75 ppmw or higher based on the total composition. The compound of formula I is optionally admixed with at least one carrier component. Carriers include (i) a copolymer of a C₂ to C₆ monoolefin; (ii) an oil soluble poly(oxyalkylene) alcohol, glycol or polyol or mono or di ether thereof; or (iii) a polyalphaolefin having a viscosity at 100°C of from 2 to 20 centistokes.

These polymeric carriers for use with the compound of formula I of the invention are individually well known in the art and patents related to their manufacture and use include, e.g., U. S. Patents Nos. 2,692,257, 2,692,258, 2,692,259, 2,918,508 and 2,970,179.

The copolymers of monoolefins which may be employed as carriers in the gasoline compositions of the invention are characterized by a number average molecular weight by osmometry preferably in the range from 500 to 1900 and more preferably in the range from 550 to 1500. Particularly preferred are those having a number average molecular weight in the range from 600 to 950. Mixtures of polymers wherein a substantial portion of the mixture has a number average molecular weight above 1500 may be less effective. The polyolefins may be prepared from unsaturated hydrocarbons having from two to six carbon atoms including, e.g., ethylene, propylene, butylene, isobutylene, butadiene, amylene, isoprene and hexene.

Preferred for their efficiency and commercial availability are copolymers of propylene and butylene; particularly preferred are polymers of isobutylene. Also suitable for use in the present invention are derivatives resulting from hydrogenation of the above polymers.

The polyoxyalkylene is the preferred carrier for use in the present invention. It can be a polyoxyalkylene compound of the formula R₃ - O - (R₄O)ₘ - R₅ wherein each of R₃ and R₅ independently represents a hydrogen atom, or an aliphatic, cycloaliphatic or aromatic hydrocarbon radical, each of which may contain up to 40 carbon atoms, R₄ represents an alkylene radical, preferably a 1,2-alkylene radical, containing up to 12 carbon atoms, and m represents an integer of at least 7, preferably at least 20 when R₄O is a 1,2-propylene group. In the polyoxyalkylene chain - (R₄O)ₘ-, the group R₄ is an alkylene radical. The polyoxyalkylene chain optionally contains two or more dissimilar alkylene groups, which preferably are alkylene radicals of 2 to 8 carbon atoms, especially ethylene or 1,2-propylene groups. These groups are optionally distributed randomly throughout the chain or arranged in a pre-determined pattern of units or blocks, each containing one or a plurality of oxyalkylene radicals.

In one embodiment of the invention, at least one of R₃ and R₅ is an alkyl or alkylphenyl group containing up to 20 carbon atoms, for example, propyl, butyl, pentyl, hexyl, octyl, nonyl, decyl, dodecyl, octylphenyl or nonylphenyl. Preferably, R₃ is hydrogen and R₅ is an alkyl group, more preferably, R₅ is dodecyl or a mixture of alkyls from C₁₂ to C₁₅.

Suitable carriers include polyoxypropylene glycols and the glycols containing both ethylene and 1,2-propylene groups in the polyoxyalkylene chain as well as the mono- and di-alkyl ethers of such glycols.

The commercially available polyoxyalkylene compounds are generally mixtures of compounds in which the values for m and the molecular weight of such mixtures are only average values. The values of m of typical compounds are usually from 7 to 100, preferably from 8 to 80. The number average molecular weights vary from 400 to 6000, preferably from 500 to 4000 and more preferably from 1000 to 2000.

When the carrier is a polyalphaolefin it has a viscosity at 100°C of from 2 centistokes to 20 centistokes. Such polyalphaolefins are suitably hydrogenated oligomers derived from alphaolefinic monomers containing at least 6 carbon atoms. The hydrogenated oligomer itself preferably contains from 18 to 80 carbon atoms. Preferably the monomer contains from 6 to 24 carbon atoms and especially 8 to 12 carbon atoms, while the oligomer preferably contains from 30 to 80 carbon atoms. The preparation of these oligomers is described in Hydrocarbon Processing, February 1982, beginning at page 75.

Mixtures, usually of equal amounts, of more than one kind of carrier can be used. The carrier is preferably a polyoxyalkylene alcohol, glycol or polyol and especially an ether thereof to help prevent low temperature intake valve sticking. Such a component is preferably used at 75 to 175 ppmw, and especially at 125 ppmw based on the total gasoline composition.

Suitable liquid hydrocarbon fuels in the gasoline boiling range are mixtures of hydrocarbons having a boiling range of from 25°C (77°F) to 232°C (450°F) and comprise mixtures of saturated hydrocarbons, olefinic hydrocarbons and aromatic hydrocarbons. Preferred are gasoline blends having a saturated hydrocarbon content ranging from 40 to 80 percent volume, an olefinic hydrocarbon content ranging from 0 to 30 percent volume and an aromatic hydrocarbon content ranging from 10 to 60 percent volume. The base fuel can be derived, for example, from straight run gasoline, polymer gasoline, natural gasoline, dimer or trimerized olefins, synthetically produced aromatic hydrocarbon mixtures from thermally or catalytically reformed hydrocarbons, or from catalytically cracked or thermally cracked petroleum stocks, or mixtures of these. The hydrocarbon composition and octane level of the base fuel are not critical. The octane level, (R+M)/2, will generally be above 85. Any conventional motor fuel base may be employed in the practice of this invention. For example, in the gasoline, hydrocarbons can be replaced by up to substantial amounts of conventional alcohols or ethers conventionally known for use in fuels. The base fuels are desirably substantially free of water, since water impedes a smooth combustion.

Preferably, the gasolines used in the present invention are lead-free, but may contain minor amounts of blending agents such as methanol, ethanol, methyl tertiary butyl ether, e.g. at from 0.1 to 15% volume of the base fuel. The gasolines may also contain antioxidants such as from 1 ppmw to 40 ppmw, based on the total gasoline composition, of phenolics, e.g., 2,6-di-tert-butylphenol, or phenylenediamines, e.g., N,N'-di-sec-butyl-p-phenylenediamine; dyes; metal deactivators; dehazers such as polyester-type ethoxylated alkylphenol-formaldehyde resins; corrosion inhibitors, such as a polyhydric alcohol ester of a succinic acid derivative having on at least one of its alpha-carbon atoms an unsubstituted or substituted aliphatic hydrocarbon group having from 20 to 500 carbon atoms, for example, pentaerythritol diester of polyisobutylene-substituted succinic acid, the polyisbutylene group having a number average molecular weight of 950, in an amount of 1 to 1000 ppmw; and antiknock compounds such as a methyl cyclopentadienylmanganese tricarbonyl or ortho-azidophenol. The additives of the present invention can be introduced into the combustion zone of the engine in a variety of ways to prevent build-up of deposits, or to accomplish reduction or modification of deposits. Thus, the additives are injected into the intake manifold intermittently or substantially continuously, preferably in a hydrocarbon carrier having a final boiling point (by ASTM D86) lower than 232°C (450°F). A preferred method is to add the additives to the gasoline. For example, the agent can be added separately to the gasoline or blended with other gasoline additives.

Hence, the present invention further provides a gasoline additive concentrate comprising a gasoline-compatible diluent and from 20 to 80%w, calculated on the diluent, of a compound of formula I as defined above.

Suitable gasoline-compatible diluents include hydrocarbons, e.g. heptane, alcohols or ethers, such as methanol, ethanol, propanol, 2-butoxyethanol or methyl tert-butyl ether. Preferably the diluent is an aromatic hydrocarbon solvent such as toluene, xylene, mixtures thereof or mixtures of tolune or xylene with an alcohol. The diluent is preferably toluene. Optionally, the concentrate may contain a dehazer, particularly a polyether-type ethoxylated alkylphenol-formaldehyde resin. The dehazer, if employed, can suitably be present in the concentrate in an amount of from 0.01 to 2%w, calculated on the diluent.

In a further aspect, the invention provides a method for operating a spark-ignition internal combustion engine which comprises introducing into the combustion chambers of said engine a gasoline composition as defined above in accordance with the invention.

In a still further aspect, the invention provides the use, as a gasoline additive for reducing intake valve deposit formation, of a compound of formula I as defined above.

Some of the compounds of formula I are known compounds whilst others are novel. Accordingly, the present invention also provides a compound of the general formula: 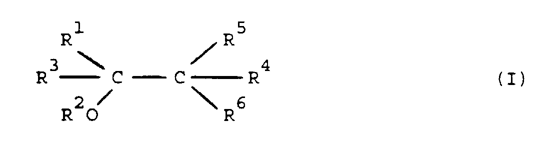 wherein R¹ represents a hydrogen atom or an alkyl, aryl or alkaryl group; R²O is a residue of a polyether mono-ol or a hydrocarbyl mono-ol of formula R²OH; and R³ and R⁴ each represent a hydrogen atom or together represent a carbon-carbon single bond, wherein when R³ and R⁴ each represent a hydrogen atom, then each of R⁵ and R⁶ independently represents a group -CH₂N(R⁷)₂ in which each R⁷ independently represents a hydrogen atom or an optionally substituted alkyl group, and when R³ and R⁴ together represent a carbon-carbon single bond, then R⁵ and R⁶ each represent a cyano group, with the proviso that when R¹ is a hydrogen atom or a C₁-C₄ alkyl group, R³ and R⁴ together represent a carbon-carbon single bond, and R⁵ and R⁶ each represent a cyano group, then R²O is a residue of a polyether mono-ol of formula R²OH.

The present invention further provides a process for the preparation of a novel compound of formula I as defined above which comprises reacting a compound of the general formula 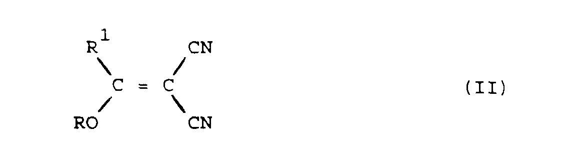 wherein R represents an alkyl group, e.g. a C₁-C₆ alkyl group, and R¹ is as defined above, with a polyether mono-ol or hydrocarbyl mono-ol of formula R²OH to produce a compound of formula I wherein R³ and R⁴ together represent a carbon-carbon single bond, followed, when R¹ is a hydrogen atom or a C₁-C₄ alkyl group and R²O is a residue of a hydrocarbyl mono-ol, and optionally followed when R²O is a residue of a polyether mono-ol or when R¹ is an alkyl group having at least 5 carbon atoms, or an aryl or alkaryl group and R²O is a residue of a hydrocarbyl mono-ol, by hydrogenating the resulting compound of formula I, optionally followed by reacting the resulting compound of formula I wherein each R⁷ represents a hydrogen atom, with a compound of the general formula

R⁷ ― L (III)

wherein L represents a leaving group, e.g. a halogen atom, and R⁷ is as defined above other than a hydrogen atom.

Reaction of a compound of formula II with R²OH is advantageously carried out at atmospheric pressure and at a temperature sufficient to maintain evolution of ROH, e.g. ethanol where R represents an ethyl group. Preferably, the temperature is in the range from 100 to 140°C. The reaction is preferably done in a batch process and reaction occurs for a time sufficient to obtain the desired amount of conversion, e.g. 4 to 5 hours. The dinitrile compound of formula II is preferably reacted with R²OH in the presence of a catalyst such as ZnCl₂.

Hydrogenation may be accomplished, for example, by reaction with a metal hydride such as aluminium hydride at atmospheric pressure and ambient temperature (20°C). Sub-ambient temperatures are preferred where the reaction is exothermic.

Alternatively, hydrogenation may be achieved by contact with hydrogen gas at elevated pressures in the presence of a hydrogenation catalyst such as a Raney cobalt or Raney nickel catalyst. Hydrogen pressures for use with such Raney catalysts are, e.g., from 2000 psi (13,790 kPa) to 3000 psi (20,684 kPa). Temperatures are preferably from 80°C to 150°C.

Reaction of a compound of formula I wherein each R⁷ represents a hydrogen atom with a compound of formula III is conveniently carried out at atmospheric pressure and at a temperature in the range from ambient temperature (20°C) to 100°C.

The compounds of formula III are known compounds or may be prepared by processes analogous to known processes.

Similarly, the compounds of formula II are known compounds or may be prepared by processes analogous to known processes, e.g. as described in GB-A-542,403, GB-A-1,018,455, GB-A-1,275,882, GB-A-1,314,801, GB-A-1,579,438 and GB-A-2,175,900.

A compound of formula II in which R¹ represents a hydrogen atom and R represents an ethyl group may conveniently be prepared by reacting equimolar quantities of malonitrile, CH₂(CN)₂, and triethyl orthoformate, (C₂H₅O)₃CH, under substantially the same reaction conditions as are used in the first reaction step of the above process, i.e. in the preparation of a compound of formula I in which R³ and R⁴ together represent a carbon-carbon single bond.

The invention will be further understood from the following illustrative examples.

### Example 1

### Preparation of 2-polybutylene oxide-1,1-dinitriloethene (a compound of formula I in which R¹ is H, R²O is a residue of polybutylene oxide (C₄H₉O(-CH₂-CH(C₂H₅)O-)ₙH), R³ and R⁴ together represent a carbon-carbon single bond and R⁵ and R⁶ each represent a cyano group)

(i) Polybutylene oxide (Mₙ 1500) obtained by the polymerisation of alpha-butylene oxide (1,2-epoxybutane) from Dow and ethoxymethylene malononitrile from Aldrich were mixed in a 1:1.02 molar ratio as a 50% xylene solution which also contained 0.08-0.2 wt% zinc chloride. The reaction temperature was maintained at a level sufficient to sustain ethanol evolution, typically from 100°C to 140°C. The reaction was complete with no further ethanol evolution after four to five hours. The reaction was allowed to cool to ambient temperature (20°C) whereupon zinc chloride was filtered off and xylene stripped to obtain solvent-free material for analysis. Analysis by Nuclear Magnetic Resonance Spectroscopy (NMR) revealed that about 100% of the polybutylene oxide was terminated with the dinitrile functional group, i.e. that the desired compound of formula I had been formed.
(ii) Step (i) was repeated except that ethoxymethylene malononitrile was replaced by equimolar quantities of malononitrile and triethyl orthoformate. The amount of ethanol evolved was increased to three equivalents per equivalent of polybutylene oxide. The desired product, 2-polybutylene oxide-1,1-dinitriloethene, was obtained.

### Example 2

### Preparation of 2-polybutylene oxide-1,1-di(methyleneamino)ethane (a compound of formula I in which R¹ is H, R²O is a residue of polybutylene oxide (C₄H₉O(-CH₂-CH(C₂H₅)O-)ₙH), R³ and R⁴ each represent a hydrogen atom and R⁵ and R⁶ each represent a group -CH₂NH₂)

1:1 (v/v) solution of 2-polybutylene oxide-1,1-dinitriloethene prepared as described in Example 1 in dry toluene was reduced with commercial Na₂AlH₂(OCH₂CH₂OCH₃)₂ (70% in toluene). Due to the exothermicity of the reaction, external cooling was employed. The product of the reaction was neutralized using an equimolar equivalent of 15% NaOH followed by quench with water. Conventional separation and drying provided the desired product.

### Example 3

The procedure of Example 2 was repeated using a 2-polybutylene oxide-1,1-dinitriloethene in which the polybutylene oxide moiety had Mₙ 1000.

### Example 4

The procedure of Example 2 was repeated using a 2-polybutylene oxide-1,1-dinitriloethene in which the polybutylene oxide moiety had Mₙ 2000.

### Example 5 - Engine Tests

Fuels with and without the compounds of Examples 1 to 4 were tested in one or both of a 3.3 liter Dodge engine with Port Fuel Injection (PFI), V-6 and a Quad-4 Oldsmobile engine with PFI for a period of 100 hours to determine the effectiveness of the compounds in reducing intake valve deposits.

The base fuel comprised premium unleaded gasoline. No carrier was used except unreacted reactants resulting from incomplete reaction during preparation of the additive. The compound of Example 1 was added to the gasoline in a concentration of 198 ppmw active matter and 2 ppmw unreacted material, primarily polybutylene oxide, R²OH, whilst the compounds of Examples 2 to 4 were each added to the gasoline in a concentration of 125 ppmw active matter, i.e., 125 ppmw diamine and some remaining unreacted material.

Each engine was in clean condition at the start of the test, i.e., oil and filters were changed and all deposits had been removed from the intake manifolds, intake ports and combustion areas of the engine. In order to test for the accumulation of deposits in the engine during each test, the engines were operated on a cycle consisting of idle mode and cruising modes of 30, 35, 45, 55 and 65 miles an hour (corresponding respectively to 48.3, 56.3, 72.4, 88.5 and 104.6 kilometres an hour) with accelerations and decelerations. The tests were conducted for 100 hours and the weight of the intake valve deposits was measured. Results of these tests are set forth in Table I below.

**Table I**

| | | Additive concentration (ppmw) | | | | Average deposit weight per valve (mg) |
|---|---|---|---|---|---|---|
| Test Run | Engine | Compound of Ex. 1 | Compound of Ex. 2 | Compound of Ex. 3 | Compound of Ex. 4 | |
| 1 | Dodge | - | - | - | - | 188 |
| | | | | | | |
| 2 | Dodge | 198 | - | - | - | 92 |
| | | | | | | |
| 3 | Dodge | - | 125 | - | - | 56 |
| | | | | | | |
| 4 | Dodge | - | - | 125 | - | 95 |
| | | | | | | |
| 5 | Dodge | - | - | - | 125 | 22 |
| | | | | | | |
| 6 | Olds | - | - | - | - | 174 |
| | | | | | | |
| 7 | Olds | 198 | - | - | - | 78 |

Results of these tests demonstrate that the gasoline compositions of the invention are very useful in very significantly preventing the accumulation of deposits in the engines tested as compared to the effects of the base fuel as shown by the much lower average valve deposits. The gasoline composition containing the compound of Example 4 showed particularly good performance.

### Example 6 - BMW Engine Test

Intake valve detergency is generally measured by the BMW NA standard of intake valve cleanliness for unlimited mileage, which is an established correlation of driveability and intake valve deposit weight of 100 milligrams or less. Intake valve deposit tests were conducted at Southwest Research Institute in 1985 model BMW 318i cars equipped with the 1.8-liter, four-cylinder engine, and were operated for 10,000 miles (16,093 kilometres) on the test fuel. Before the test started, deposits were removed from the cylinder head, intake manifold and piston tops and new intake valves were weighed and installed. The oil and filter were changed, new spark plugs installed and the fuel injectors flow checked. Mileage was accumulated on public roads using trained drivers. The test route consisted of about 10% city driving, 20% on secondary roads and 70% highway driving (maximum speed of 65 mph (104.6 km/h)).

The primary test data are the intake valve deposit (IVD) weights at the end of the 10,000-mile (16,093 km) test. IVD weights are also determined at 5,000 miles (8,047 km), where tests can be terminated if the results are not promising. BMW's pass criteria are as follows: an average deposit weight of 100 milligrams per valve or less at the conclusion of the test meets BMW requirements for unlimited mileage acceptance; and an average deposit weight of 250 mg per valve or less at the conclusion of the test meets BMW requirements for 50,000-mile (80,467 km) service.

Premium unleaded base gasoline containing the compound of Example 4 at a concentration of 172 ppmw active matter was tested in the BMW engine test. The average intake valve deposit weight at the end of the test (10,000 miles/16,093 km) was 46 mg. Thus, the result of this test demonstrates that the gasoline composition of the invention passes the BMW unlimited mileage test with low deposits.

## Claims

1. A gasoline composition comprising a major amount of a gasoline and a minor amount of a compound of the general formula: 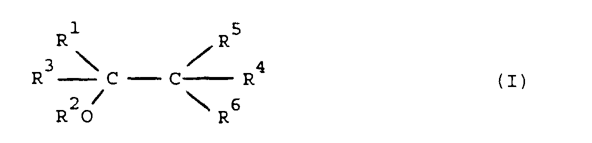 wherein R¹ represents a hydrogen atom or an alkyl, aryl or alkaryl group; R²O is a residue of a polyether mono-ol or a hydrocarbyl mono-ol of formula R²OH; and R³ and R⁴ each represent a hydrogen atom or together represent a carbon-carbon single bond, wherein when R³ and R⁴ each represent a hydrogen atom, then each of R⁵ and R⁶ independently represents a group -CH₂N(R⁷)₂ in which each R⁷ independently represents a hydrogen atom or an optionally substituted alkyl group, and when R³ and R⁴ together represent a carbon-carbon single bond, then R⁵ and R⁶ each represent a cyano group.

2. A gasoline composition according to claim 1, wherein R³ and R⁴ each represent a hydrogen atom, and each of R⁵ and R⁶ independently represents a group -CH₂N(R⁷)₂ as defined in claim 1.

3. A gasoline composition according to claim 2, wherein each R⁷ independently represents a hydrogen atom or a C₁-C₂₀ alkyl group.

4. A gasoline composition according to any one of claims 1 to 3, wherein R¹ represents a hydrogen atom, a C₁-C₂₀ alkyl group or a phenyl or naphthyl group optionally substituted by one or more C₁-C₆ alkyl groups.

5. A gasoline composition according to any one of claims 1 to 4, wherein R²OH is an oil soluble polyether mono-ol of the formula R₁-O-(R₂O)ₙ-H wherein R₁ represents an aliphatic, cycloaliphatic or aromatic hydrocarbon radical containing up to 40 carbon atoms, each moiety R₂, which may be the same or different, represents an alkylene radical containing up to 16 carbon atoms, and n is an integer of at least 7.

6. A gasoline composition according to any one of claims 1 to 5, wherein R²OH is of the formula R₁O-(EO)ₐ-(PO)_{b}-(BO)_{c}-(XO)_{d}-H wherein R₁ represents an aliphatic, cycloaliphatic or aromatic hydrocarbon radical containing up to 40 carbon atoms, and wherein E is an ethylene moiety, P is a 1,2-propylene moiety, B is a butylene moiety, X is an alkylene moiety having from 5 to 16 carbon atoms; a, b, c and d are each from 0 to 100, and the sum of a, b, c and d is at least 7.

7. A gasoline additive concentrate comprising a gasoline-compatible diluent and from 20 to 80%w, calculated on the diluent, of a compound of formula I as defined in any one of the preceding claims.

8. Use, as a gasoline additive for reducing intake valve deposit formation, of a compound of formula I as defined in any one of claims 1 to 6.

9. A compound of the general formula: 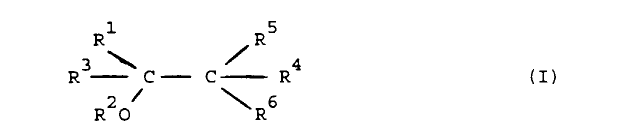 wherein R¹ represents a hydrogen atom or an alkyl, aryl or alkaryl group; R²O is a residue of a polyether mono-ol or a hydrocarbyl mono-ol of formula R²OH; and R³ and R⁴ each represent a hydrogen atom or together represent a carbon-carbon single bond, wherein when R³ and R⁴ each represent a hydrogen atom, then each of R⁵ and R⁶ independently represents a group -CH₂N(R⁷)₂ in which each R⁷ independently represents a hydrogen atom or an optionally substituted alkyl group, and when R³ and R⁴ together represent a carbon-carbon single bond, then R⁵ and R⁶ each represent a cyano group, with the proviso that when R¹ is a hydrogen atom or a C₁-C₄ alkyl group, R³ and R⁴ together represent a carbon-carbon single bond, and R⁵ and R⁶ each represent a cyano group, then R²O is a residue of a polyether mono-ol of formula R²OH.

10. A process for the preparation of a compound of formula I as defined in claim 9, which comprises reacting a compound of the general formula 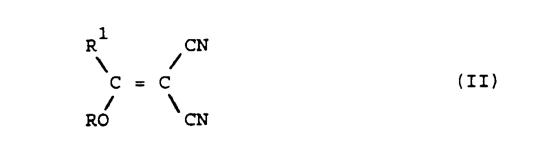 wherein R represents an alkyl group and R¹ is as defined in claim 9, with a polyether mono-ol or hydrocarbyl mono-ol of formula R²OH to produce a compound of formula I wherein R³ and R⁴ together represent a carbon-carbon single bond, followed, when R¹ is a hydrogen atom or a C₁-C₄ alkyl group and R²O is a residue of a hydrocarbyl mono-ol, and optionally followed when R²O is a residue of a polyether mono-ol or when R¹ is an alkyl group having at least 5 carbon atoms, or an aryl or alkaryl group and R²O is a residue of a hydrocarbyl mono-ol, by hydrogenating the resulting compound of formula I, optionally followed by reacting the resulting compound of formula I wherein each R⁷ represents a hydrogen atom, with a compound of the general formula
R⁷ ― L (III)
wherein L represents a leaving group and R⁷ is as defined in claim 9 other than a hydrogen atom.

## Patentansprüche

1. Benzinzusammensetzung, umfassend eine Hauptmenge an einem Benzin und eine kleine Menge an einer Verbindung mit der allgemeinen Formel: 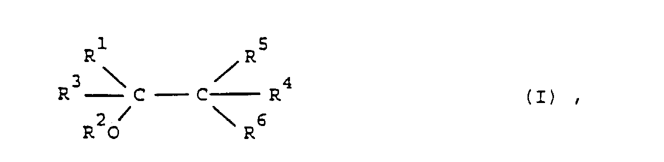 worin R¹ ein Wasserstoffatom oder eine Alkyl-, Aryl- oder Alkarylgruppe darstellt, R²O ein Rest eines Polyethermonools oder eines Hydrocarbylmonools mit der Formel R²OH ist; und R³ und R⁴ jeweils ein Wasserstoffatom darstellen oder gemeinsam eine Kohlenstoff-Kohlenstoff-Einfachbindung bedeuten, wobei dann, wenn R³ und R⁴ jeweils ein Wasserstoffatom bedeuten, jeder der Reste R⁵ und R⁶ unabhängig für eine Gruppe -CH₂N(R⁷)₂ steht, worin jeder Rest R⁷ unabhängig ein Wasserstoffatom oder eine gegebenenfalls substituierte Alkylgruppe darstellt, und dann, wenn R³ und R⁴ gemeinsam eine Kohlenstoff-Kohlenstoff-Einfachbindung bedeuten, die Reste R⁵ und R⁶ jeweils eine Cyanogruppe darstellen.

2. Benzinzusammensetzung nach Anspruch 1, worin R³ und R⁴ jeweils ein Wasserstoffatom darstellen und jeder Rest R⁵ und R⁶ unabhängig eine Gruppe -CH₂N(R⁷)₂, wie im Anspruch 1 definiert, darstellt.

3. Benzinzusammensetzung nach Anspruch 2, worin jeder Rest R⁷ unabhängig ein Wasserstoffatom oder eine C₁-C₂₀-Alkylgruppe darstellt.

4. Benzinzusammensetzung nach einem der Ansprüche 1 bis 3, worin R¹ ein Wasserstoffatom, eine C₁-C₂₀-Alkylgruppe oder Phenyl- oder Naphthylgruppe darstellt, die gegebenenfalls durch eine oder mehrere C₁-C₆-Alkylgruppen substituiert sind.

5. Benzinzusammensetzung nach einem der Ansprüche 1 bis 4, worin R²OH ein öllösliches Polyethermonool mit der Formel R₁-O-(R₂O)ₙ-H ist, worin R₁ einen aliphatischen, cycloaliphatischen oder aromatischen Kohlenwasserstoffrest mit bis zu 40 Kohlenstoffatomen bedeutet, jeder Rest R₂, der gleich oder verschieden sein kann, einen Alkylenrest mit bis zu 16 Kohlenstoffatomen bedeutet und n eine ganze Zahl von wenigstens 7 ist.

6. Benzinzusammensetzung nach einem der Ansprüche 1 bis 5, worin R²OH der Formel R₁O-(EO)ₐ-(PO)_{b}-(BO)_{c}-(XO)_{d}-H entspricht, worin R₁ einen aliphatischen, cycloaliphatischen oder aromatischen Kohlenwasserstoffrest mit bis zu 40 Kohlenstoffatomen darstellt und worin E einen Ethylenrest bedeutet, P ein 1,2-Propylenrest ist, B ein Butylenrest ist, X einen Alkylenrest mit 5 bis 16 Kohlenstoffatomen bezeichnet; a, b, c und d jeweils von 0 bis 100 bedeuten und die Summe aus a, b, c und d wenigstens 7 beträgt.

7. Benzinadditivkonzentrat, umfassend ein benzinverträgliches Verdünnungsmittel und 20 bis 80 Gew.-%, bezogen auf das Verdünnungsmittel, einer Verbindung der Formel I, wie in einem der vorstehenden Ansprüche definiert.

8. Verwendung einer Verbindung der Formel I, wo in einem der Ansprüche 1 bis 6 definiert, als Benzinadditiv zur Verringerung der Ausbildung von Einlaßventil-Ablagerungen.

9. Verbindung mit der allgemeinen Formel: 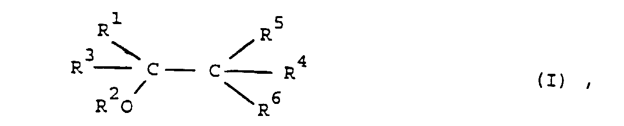 worin R₁ ein Wasserstoffatom oder eine Alkyl-, Aryl- oder Alkarylgruppe darstellt, R²O ein Rest eines Polyethermonools oder eines Hydrocarbylmonools mit der Formel R²OH ist; und R³ und R⁴ jeweils ein Wasserstoffatom darstellen oder gemeinsam eine Kohlenstoff-Kohlenstoff-Einfachbindung bedeuten, wobei dann, wenn R³ und R⁴ jeweils ein Wasserstoffatom bedeuten, jeder der Reste R⁵ und R⁶ unabhängig für eine Gruppe -CH₂N(R⁷)₂ steht, worin jeder Rest R⁷ unabhängig ein Wasserstoffatom oder eine gegebenenfalls substituierte Alkylgruppe darstellt, und dann, wenn R³ und R⁴ gemeinsam eine Kohlenstoff-Kohlenstoff-Einfachbindung bedeuten, die Reste R⁵ und R⁶ jeweils eine Cyanogruppe darstellen, mit der Maßgabe, daß dann, wenn R₁ ein Wasserstoffatom oder eine C₁-C₄-Alkylgruppe ist, R³ und R⁴ zusammen eine Kohlenstoff-Kohlenstoff-Einfachbindung darstellen und R⁵ und R⁶ jeweils eine Cyanogruppe bedeuten, R²O einen Rest eines Polyethermonools mit der Formel R²OH darstellt.

10. Verfahren zur Herstellung einer Verbindung der Formel I, wie in Anspruch 9 definiert, welches ein Umsetzen einer Verbindung der allgemeinen Formel 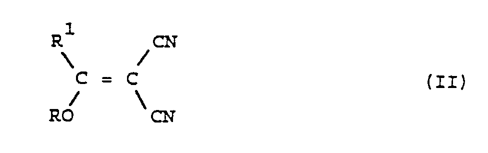 worin R eine Alkylgruppe darstellt und R₁ wie im Anspruch 9 definiert ist, mit einem Polyethermonool oder Hydrocarbylmonool der Formel R²OH zur Ausbildung einer Verbindung der Formel I, worin R³ und R⁴ zusammen eine Kohlenstoff-Kohlenstoff-Einfachbindung darstellen, gefolgt von einer Hydrierung der erhaltenen Verbindung der Formel I, wenn R₁ ein Wasserstoffatom oder eine C₁-C₄-Alkylgruppe ist und R²O ein Rest eines Hydrocarbylmonools ist, und gegebenenfalls gefolgt von einer Hydrierung der erhaltenen Verbindung der Formel I, wenn R²O ein Rest eines Polyethermonools ist oder wenn R₁ eine Alkylgruppe mit wenigstens 5 Kohlenstoffatomen oder eine Aryl- oder Alkarylgruppe ist und R²O ein Rest eines Hydrocarbylmonools ist, gegebenenfalls gefolgt von einem Umsetzen der erhaltenen Verbindung der Formel I, worin jeder Rest R⁷ ein Wasserstoffatom bedeutet, mit einer Verbindung der allgemeinen Formel
R⁷-L (III)
umfaßt, worin L eine Leaving-Gruppe darstellt und R⁷ wie im Anspruch 9 definiert ist, jedoch eine andere Bedeutung als ein Wasserstoffatom hat.

## Revendications

1. Composition d'essence comprenant une quantité majeure d'une essence et une quantité mineure d'un composé de la formule générale : 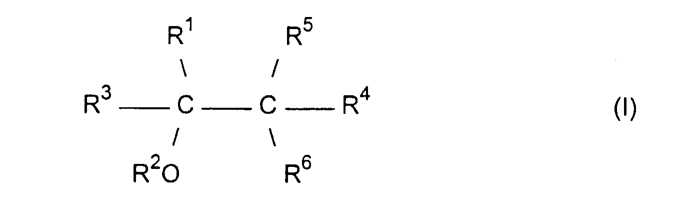 dans laquelle R¹ représente un atome d'hydrogène ou un radical alkyle, aryle ou alcaryle; R²O représente le reste d'un polyéther monool ou d'un hydrocarbylmonool de la formule R²OH; et R³ et R⁴ représentent chacun un atome d'hydrogène ou représentent ensemble une simple liaison carbone à carbone, où lorsque R³ et R⁴ représentent chacun un atome d'hydrogène, alors chacun des symboles R⁵ et R⁶ représente indépendamment un groupe -CH₂N(R⁷)₂ dans lequel chaque symbole R⁷ représente indépendamment un atome d'hydrogène ou un groupe alkyle éventuellement substitué et lorsque R³ et R⁴ représentent ensemble une simple liaison carbone à carbone, alors R⁵ et R⁶ représentent chacun un radical cyano.

2. Composition suivant la revendication 1, caractérisée en ce que R³ et R⁴ représentent chacun un atome d'hydrogène et chacun des symboles R⁵ et R⁶ représente indépendamment un groupe -CH₂N(R⁷)₂, tel que défini dans la revendication 1.

3. Composition d'essence suivant la revendication 2, caractérisée en ce que chaque symbole R⁷ représente indépendamment un atome d'hydrogène ou un radical alkyle en C₁ à C₂₀.

4. Composition d'essence suivant l'une quelconque des revendications 1 à 3, caractérisée en ce que R₁ représente un atome d'hydrogène, un radical alkyle en C₁ à C₂₀, ou un radical phényle ou naphtyle éventuellement substitué par un ou plusieurs radicaux alkyle en C₁ à C₆.

5. Composition d'essence suivant l'une quelconque des revendications 1 à 4, caractérisée en ce que R²OH est un polyéther monool oléosoluble de la formule R₁-O-(R₂O)ₙ-H dans laquelle R₁ représente un radical hydrocarboné aliphatique, cycloaliphatique ou aromatique contenant jusqu'à 40 atomes de carbone, chaque groupement R₂, pouvant être identique ou différent, représente un radical alkylène contenant jusqu'à 16 atomes de carbone et n est un nombre entier égal à au moins 7.

6. Composition d'essence suivant l'une quelconque des revendications 1 à 5, caractérisée en ce que R²OH répond à la formule R₁O-(EO)ₐ-(PO)_{b}-(BO)_{c}-(XO)_{d}-H dans laquelle R₁ représente un radical hydrocarboné aliphatique, cycloaliphatique, ou aromatique, contenant jusqu'à 40 atomes de carbone et dans laquelle E représente un groupement éthylène, P représente un groupement 1,2-propylène, B représente un groupement butylène, X représente un groupement alkylène comportant de 5 à 16 atomes de carbone, a, b, c, et d varient chacun de 0 à 100 et la somme de a, b, c et d est au moins égale à 7.

7. Concentré d'additif pour essence comprenant un diluant compatible avec l'essence et de 20 à 80% en poids, calculés sur le diluant, d'un composé de la formule 1, tel que défini dans l'une quelconque des revendications précédentes.

8. Utilisation, à titre d'additif pour l'essence destiné à réduire la formation de dépôts sur les soupapes d'admission, d'un composé de la formule 1, tel que défini dans l'une quelconque des revendications 1 à 6.

9. Composé de la formule générale: 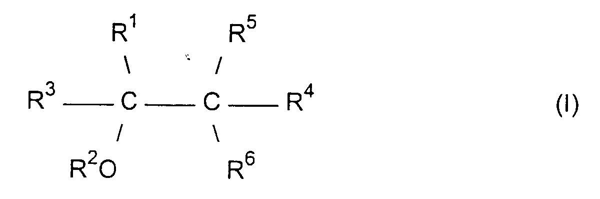 dans laquelle R¹ représente un atome d'hydrogène ou un radical alkyle, aryle ou alcaryle; R²O représente le reste d'un polyéther monool ou d'un hydrocarbylmonool de la formule R²OH; et R³ et R⁴ représentent chacun un atome d'hydrogène ou représentent ensemble une simple liaison carbone à carbone, où lorsque R³ et R⁴ représentent chacun un atome d'hydrogène, alors chacun des symboles R⁵ et R⁶ représente indépendamment un groupe -CH₂N(R⁷)₂ dans lequel chaque symbole R⁷ représente indépendamment un atome d'hydrogène ou un groupe alkyle éventuellement substitué et lorsque R³ et R⁴ représentent ensemble une simple liaison carbone à carbone, alors R⁵ et R⁶ représentent chacun un radical cyano, avec la condition que lorsque R¹ représente un atome d'hydrogène ou un groupe alkyle en C₁ à C₄, R³ et R⁴ représentent ensemble une simple liaison carbone à carbone et que lorsque R⁵ et R⁶ représentent chacun un groupe cyano, alors R²O représente le reste d'un polyéther monool de la formule R²OH.

10. Procédé de préparation d'un composé de la formule I, tel que défini dans la revendication 9, caractérisé en ce que l'on fait réagir un composé de la formule générale 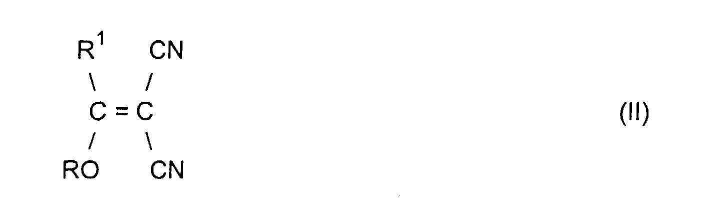 dans laquelle R représente un groupe alkyle et R¹ est tel que défini dans la revendication 9, avec un polyéther monool ou un hydrocarbylmonool de la formule R²OH de manière à produire un composé de la formule I dans laquelle R³ et R⁴ représentent ensemble une simple liaison carbone à carbone, réaction suivie, lorsque R¹ représente un atome d'hydrogène ou un radical alkyle en C₁ à C₄ et R²O est le reste d'un hydrocarbylmonool et éventuellement suivie, lorsque R²O est un reste d'un polyéther monool ou lorsque R¹ est un radical alkyle possédant au moins 5 atomes de carbone, ou un radical aryle ou alcaryle et R²O est un reste d'un hydrocarbylmonool, de l'hydrogénation du composé résultant de la formule I, éventuellement suivie de la réaction du composé résultant de la formule I dans laquelle chaque symbole R⁷ représente un atome d'hydrogène, avec un composé de la formule générale
R⁷ ― L (III)
dans laquelle L représente un groupe sortant ou labile et R⁷ est tel que défini dans la revendication 9, mais diffère d'un atome d'hydrogène.
